# EUROPEAN PATENT SPECIFICATION

(11) **EP 3 197 399 B1**
(45) Date of publication and mention of the grant of the patent: **01.04.2020**
(21) Application number: 15775480.5
(22) Date of filing: 23.09.2015
(51) Int. Cl.: A61F 2/28, A61B 17/68, A61B 17/56, A61F 2/30

(54) **DEVICE AND METHOD OF ITS FABRICATION**
VORRICHTUNG UND VERFAHREN ZU DEREN HERSTELLUNG
APPAREIL ET METHODE DE FABRICATION

(30) Priority: 23.09.2014 GB 201416790
(43) Date of publication of application: 02.08.2017
(73) Proprietor: UCL BUSINESS LTD, London W1T 4TP (GB)
(72) Inventor: DUNAWAY, David, London Greater London W1T 4TP (GB); BORGHI, Alessandro, London Greater London W1T 4TP (GB); JEELANI, Owase, London Greater London W1T 4TP (GB); SCHIEVANO, Silvia, London Greater London W1T 4TP (GB)
(74) Representative: Jones, Nicholas Andrew
(86) International application number: PCT/GB2015/052758
(87) International publication number: WO 2016/046549

(56) References cited:
- WO-A1-2012/036129
- WO-A1-2013/154697
- WO-A2-2014/125381
- US-A- 5 776 195
- US-A1- 2004 249 461
- US-A1- 2014 088 618

## Description

The present invention relates to a device, method and kit. In particular, though not exclusively, it concerns a medical device comprising a shape memory material for the distraction of bone in 3 dimensions, methods of preparing the device, and uses thereof.

The need to lengthen and remodel the shapes of tissues and/or bones poses many difficult challenges, particularly in the areas of orthopaedic and cranio-maxillofacial reconstruction.

Starting in the 1970s, Ilizarov (The principles of the Ilizarov method. Bull. Hosp. Jt. Dis. Orthop. Inst. 48: 1, 1988) described a process of distraction osteogenesis to lengthen the long bones in limbs. The process involved weakening the bone to be lengthened by performing corticotomies (cuts in the outer layer of the bone). An external adjustable framework was then used to distract the bone across the corticotomy. Bone lengthening occurs as callus is formed in response to the microfractures that occur in response to the distracting force.

Subsequently, McCarthy (Lengthening the human mandible by gradual distraction. Plast. Reconstr. Surg. 103: 1592, 1999) introduced more sophisticated devices that could lengthen bones in the craniofacial skeleton and since then ever more complex distractors have been developed. More recently, Lauritzen (Spring-assisted cranioplasty vs pi-plasty for sagittal synostosis - A Long Term Follow-Up Study. The Journal Of Craniofacial Surgery, 19: 1 2008) introduced a technique using implantable springs that could be used to gradually mould skull bones of babies with misshapen skulls.

In particular, children with syndromic craniosynostosis suffer the premature fusion of several cranial sutures which restricts the growth of the skull and face. The resultant disturbances of growth, along with other effects resulting from the expression of an abnormal gene, lead to deformity and several functional problems. Restriction of skull growth may result in raised intracranial pressure, whilst failure of facial growth may result in lack of eye protection, upper airway obstruction and feeding and speech difficulties.

The growth disturbances are present at birth and tend to be progressive throughout childhood. Treatment involves regular surveillance from infancy to adulthood by a dedicated multidisciplinary team skilled in managing the functional pathologies and anatomical anomalies that may arise.

Frontofacial distraction has proved functionally and aesthetically effective in treating the deformities caused by craniosynostosis, but remains a major procedure with a 1% mortality rate and approximately 10% major complication rate. The final outcome also remains moderately unpredictable because of an incomplete understanding of normal craniofacial surgery anatomy and unsophisticated distractor design.

Thus, while all these techniques have proven effective to a certain extent, they lack precision and have no pre-determined endpoint.

In particular, US 6,908,467 discloses a fixation device for internally or externally fixing fractures comprising at least one nitinol wire having an S-shaped section and two ends wherein each of the two ends forms a hook for hooking into a bone section of a fractured bone. The nitinol wire elongates in a longitudinal direction to generate a distraction force.

Similarly, Zhou et al. (Journal of Craniofacial Surgery, Vol. 17, Issue 5, 2006, Pages 943-949) discloses a form of transport distraction osteogenesis using a nitinol spring. Simple devices, including internal 60 mm long sinusoid-shaped nitinol springs, were used in this study. study.

Document WO2013/154697 discloses a a device for distracting bone according to the preamble of claim 1.

However, all devices in the prior art have only been able to distract bone in a single direction or dimension. More complex re-shaping of bone structures has so far been impossible, with reconstructions using bone fragments from other bodily sites being the default methodology. The inventors have therefore devised a novel device and technique that alters, using shape memory materials, bone shape and size by callus distraction and/or bone shape and size by gradual moulding. The device may be used internally and/or externally of the body of the subject to be treated.

Accordingly, in a first aspect of the invention, there is provided a device for distracting bone, the device comprising a shape memory material and being capable of distracting bone in 3 dimensions, wherein the shape memory material is nitinol and is a continuous sheet, mesh or web, and wherein the shape memory material is arranged into a predetermined 3-dimensional conformation in the austenitic phase of nitinol which corresponds to the desired shape of the bone to be distracted. Such a device has been found to deliver the ability to precisely remodel bones to a desired morphology. The device is typically individually tailored to each subject to be treated, and allows the matter being modulated to be reshaped in a very controlled manner.

As used herein, the term "shape memory material" refers to a material that "remembers" its original, pre-programmed shape, such that following deformation it returns to its pre-deformed shape upon application of an external stimulus (such as a change in temperature).

The ability of the shape memory material to return to its original, pre-programmed shape therefore allows the device to distract bone adjacent thereto simultaneously in 3 dimensions. This means that whereas the prior art has concerned lengthening bone in one dimension only, the present invention facilitates the reconstruction of the bone concerned to its complete original shape.
The physical arrangement of the device intended for use is dependent on the shape of the bone to be modulated. It is preferable, nevertheless, that the shape memory material is arranged into a predetermined 3-dimensional conformation upon application of an external stimulus (e.g. elevated temperature). In other words, the shape memory material is first set (typically using a mould) in a shape corresponding to the final, desired shape of the bone to be modulated, under application of the external stimulus. The application of the external stimulus causes the shape memory material to form a specific internal structure which facilitates its ability to "remember" the desired shape.

Following setting of the memory shape, the material is then moulded to the present conformation of the bone to be treated under conditions in which there is no application of the external stimulus. In the absence of the external stimulus, the internal structure of the shape memory material transforms into a malleable form which can be readily deformed into the present conformation of the bone.

In use, further application of the external stimulus causes the shape memory material to deform back to its shape memory internal structure, and thus the original, predetermined 3-dimensional conformation, i.e. the final, desired shape of the bone. Such an arrangement is preferably a 3-dimensional arrangement in nature and provides a modulating/distracting force in 3-dimensions. This arrangement is therefore effective in reshaping bone, or directing bone growth or development.

In a preferred embodiment of the invention, the shape memory material may be a continuous sheet arranged in a predetermined 3-dimensional conformation. The continuous sheet may be a flat piece of shape memory material, optionally containing essentially no holes in the body of the sheet, and which has been moulded to a specific 3-dimensional shape for application in the distraction or bone.

In an alternative preferred embodiment, the shape memory material may be a mesh or web arranged in a predetermined 3-dimensional conformation. In this embodiment, the 3-dimensional construct may have a series of connected strands of the material which form a mesh-, web- or net-type structure. Alternatively, and preferably from the point of view of ease of construction, the mesh, web or net may be a sheet of memory material in which one or more, and preferably a plurality of holes have been created (e.g. by laser cutting techniques).

In particular, the mesh or web may comprise a network of geometric shapes, such as circular, triangular, square, pentagonal, or hexagonal shapes, or a combination thereof. For example, the mesh or web may be provided by a plurality of holes, comprising any of the above shapes (preferably circular or hexagonal shapes), which have been created through a continuous sheet of the memory shape material. Preferably, the mesh or web is provided by a plurality of essentially circular-shaped holes in a sheet of the memory shape material. By using a mesh, it has been surprisingly found that the device can provide much more control over bone growth and distraction and allow the correction of much more complicated bone structures.

The average thickness of the sheet, mesh or web is preferably less than 5 mm, 4 mm, or 3 mm. More preferably, it is less than 2 mm or 1 mm. Most preferably, the average thickness of the sheet, mesh or web is in the range of 0.2 mm to 1 mm, since this thickness provides the optimum performance in terms of strength and flexibility.

The physical composition of the shape memory material is nitinol, a shape memory alloy.

The shape-memory alloy (often alternatively referred to in the art as a smart metal, memory metal, memory alloy, or smart alloy) is a smart material that has the ability to return from a deformed state (temporary shape) to their original (permanent) shape when induced by an external stimulus (trigger), such as a temperature change.

Nitinol (a nickel-titanium-based shape memory alloy) is used due to its stability, practicability and superior thermo-mechanical performance.

Nickel-titanium, also known as nitinol, is a metal alloy of nickel and titanium, where the two elements are present in roughly equal atomic percentages, e.g. Nitinol 55, Nitinol 60. Nitinol is preferred in the context of the present invention as it is highly biocompatible and has properties suitable for use in orthopaedic implants.

Furthermore, nitinol alloys exhibit two closely related and unique properties: shape memory and superelasticity (also called pseudoelasticity). Shape memory is the ability of nitinol to undergo deformation at one temperature, then recover its original, undeformed shape upon heating above its "transformation temperature". Superelasticity occurs at a narrow temperature range just above its transformation temperature; in this case, no heating is necessary to cause the undeformed shape to recover, and the material exhibits enormous elasticity, some 10-30 times that of ordinary metal.

In addition, the phase transformation exhibited by nitinol is "reversible", meaning that heating above the transformation temperature will revert the crystal structure to the simpler austenite phase. Another key feature is that the transformation in both directions is instantaneous.

At high temperatures (e.g. at and above body temperature of approximately 37 °C), the nitinol for use in the invention assumes an interpenetrating primitive cubic crystal structure referred to as austenite (also known as the parent phase). At low temperatures (e.g. near to and below body temperature of approximately 37 °C), the nitinol spontaneously transforms to a more complicated monoclinic crystal structure known as martensite (daughter phase). The temperature at which austenite transforms to martensite is generally referred to as the transformation or transition temperature. When the alloy is fully austenite, martensite begins to form as the alloy cools at the so-called martensite start, or Mₛ temperature, and the temperature at which the transformation is complete is called the martensite finish, or M_{f} temperature. When the alloy is fully martensite and is subjected to heating, austenite starts to form at the Aₛ temperature, and finishes at the A_{f} temperature.

In the present invention, the transition from martensite to austenite preferably occurs at approximately body temperature (i.e. at about 37 °C). This means that the return of the device from its deformed shape back to its original, pre-programmed shape may be triggered simply by use of the device with a human subject. In this case, the working temperature is body temperature.

Martensite's crystal structure has the unique ability to undergo limited deformation in some ways without breaking atomic bonds. This type of deformation is known as twinning, which consists of the rearrangement of atomic planes without causing slip, or permanent deformation. It is able to undergo about 6-8% strain in this manner. When martensite is reverted to austenite by heating, the original austenitic structure is restored, regardless of whether the martensite phase was deformed. Thus, the name "shape memory" refers to the fact that the shape of the high temperature austenite phase is "remembered", even if the alloy is severely deformed at a lower temperature.

One of the possible reasons that nitinol works so hard to return its original shape is that it is not just an ordinary metal alloy, but is what is known as an intermetallic compound. In an ordinary alloy, the constituents are randomly positioned in the crystal lattice, whereas in an ordered intermetallic compound, the atoms (in this case, nickel and titanium) have very specific locations in the lattice. This means that a large degree of force can be produced by preventing the reversion of deformed martensite to austenite, such as from 240MPa (35,000 psi) to, in many cases, more than 689 MPa (100,000 psi).

Nitinol is typically composed of approximately 40 to 60% nickel by atomic percent, preferably 50 to 60% nickel by atomic percent, more preferably 52 to 58% nickel by atomic percent. The A_{f} temperature can be controlled in nitinol to some extent depending on the content of nickel and titanium. The invention is effective when the A_{f} temperature is below the working temperature. Convenient working temperature ranges are from about -20 °C to 60 °C, preferably 0 °C to 50 °C, more preferably 5 °C to 40 °C or 30 °C to 40 °C (e.g. body temperature of approximately 37 °C). At such temperatures, nitinol displays hyperelastic properties.

In another aspect of the invention, there is provided a device according to the invention, for use in bone distraction. The device is useful in the distraction of bone from one existing conformation into another, pre-determined conformation.

More specifically, suitable uses include dentistry and oral and maxillofacial surgery applications, the treatment of craniosynostosis or other orthopaedic abnormalities or traumas.

For example, in dentistry the device may be used in orthodontics for constructs connecting the teeth. Once the shape memory material is placed in the mouth, its temperature rises to ambient body temperature. This causes the material to contract back to its original shape, applying a constant force to move the teeth. Advantageously, such constructs do not need to be retightened as often as conventional stainless steel wires.

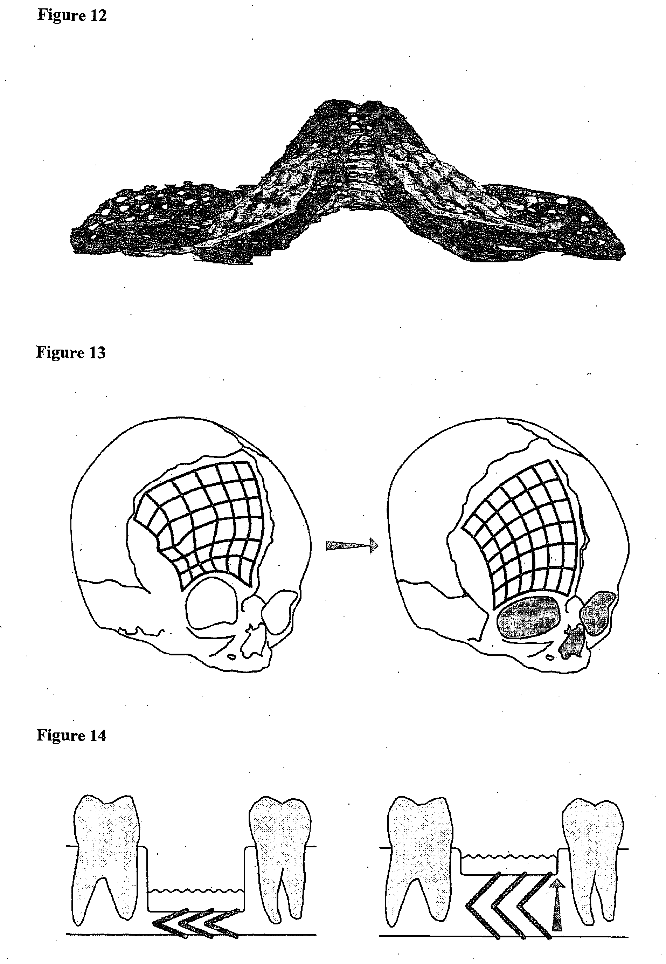

Intraoral distraction to modify bone stock for implant insertion is also a suitable application of the device of the invention. For example, a common problem in long-standing edentulous segments is to find enough bone in the correct position to place dental implants for dental reconstruction. The invention may therefore be used to expand and distract bone to a desired level (e.g. see Figure 14).

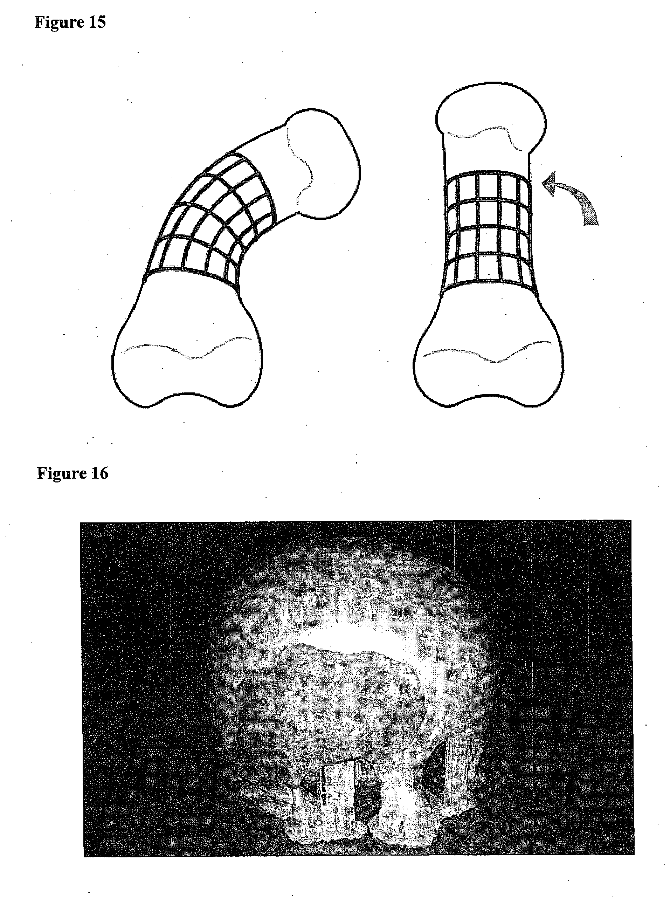

In terms of treating craniosynostosis, the device can be used to remodel a twisted skull, or correct a flattened forehead (e.g. see Figure 13). Of particular interest, the invention may be used to remodel craniofacial bones deformed by congenital anomalies or trauma. Similarly, the remodelling of other bones, such as in the hands and feet, may involve the realignment, lengthening or reshaping of the existing 3-dimensional conformation (e.g. see Figure 15).

In relation to engineering constructs for reconstructive surgery, free tissue transfer is commonly used to reconstruct areas of the body damaged by injury or tumour or where there has been a failure of normal development. Bone, skin, muscle and other organs may be harvested from less vital areas of the body and used as materials for reconstruction. However, one major problem with this technique is that the bone and skin available may not be of the required shape and/or size.

This is the case, for example, where part of the mandible has been removed to treat a particular condition. A piece of hip bone (ileac crest) may be used for the reconstruction, but is rarely of the correct shape. A new piece of mandible may be designed to replace the bone removed and a best fit found on the hip. However, there is no exact match. Thus, the device of the invention can be used to deform donor area bone as described above before transfer to the recipient area to be reconstructed. This produces a more exact match to the desired bone shape.

The present invention is therefore applicable in the treatment of damaged or deformed bone resulting from musculoskeletal trauma, sports injuries, degenerative diseases, infections, tumors, and congenital disorders. In particular, the invention is useful in calvarial remodeling, including posterior vault expansion, craniosynostosis (including unicoronal synostosis) and/or sagittal synostosis.

In another aspect of the invention, there is provided a process for producing a device for distracting bone, the process comprising: (i) determining the current and desired conformations of the bone; (ii) shaping a device comprising a shape memory material into the desired conformation at a temperature around or above body temperature; and (iii) moulding the device into the current bone conformation at a temperature below body temperature.

It will be appreciated that any of the features mentioned above in relation to the device of the invention are also applicable to the method of producing the device.

The determination of the current conformation of the bone may be conducted using any analytical medical method and/or device, such as X-ray, magnetic resonance imaging, computed tomography, laser surface scanning and 3D photogrammetry. In particular, it has been found that computed tomography (CT) provides accurate results in determining the present conformation of the bone.

The determination of the desired conformation of the bone may be conducted using any suitable approach, such as a computer modelling method. There are several commercially available reconstructive modelling programs available (e.g. those available from Materialise®) Geometric morphometric analysis using a principal component analysis-derived, computer-generated template may also be used (Dunaway et al., Planning surgical reconstruction in treacher-collins syndrome using virtual simulation. Plast. Reconstr. Surg. 2013 Nov; 132(5): 790e-805e). Templates or moulds may also be constructed from physical adaptation of steriolithographic models printed from CT or other 3D imaging modalities.

The shaping of the device into the desired, pre-programmed conformation is usually conducted at a temperature above body temperature. Generally, this is achieved at a temperature between the forming temperature of the shape memory material and slightly below the melting point of the shape memory material. For example, in the case of nitinol, depending on the specific composition employed, shaping of the device may be conducted at a temperature from 300 °C to 1300 °C, preferably 400 °C to 1250 °C, more preferably 500 °C to 1200 °C.

The moulding of the device into the current bone conformation is carried out at a temperature below body temperature, i.e. below 37 °C, 36 °C, or 35 °C. In a preferred embodiment, the moulding is conducted at a temperature between 0 and 34 °C.

In particular, the process of cooling austenite to form martensite, deforming the martensite, then heating to revert to austenite, thus returning the original, undeformed shape is known as the thermal shape memory effect. To fix the original "parent shape", the material can be held in position in a preformed mould constructed from a material with high thermal conductivity to allow rapid and even heating of the shape memory material, then heated to above about 300 °C, 400 °C, or 450 °C, preferably to about 500 °C (932 °F). In a certain temperature range, such as below 34 °C, austenite can be transformed into martensite, while at the same time changing its shape. In this case, as soon as the stress is removed, and upon raising of the temperature to approximately body temperature, i.e. at or above approximately 37 °C, the martensite spontaneously returns to its original shape in the austenite form. In this way, material (preferably nitinol) behaves like a super spring, possessing an elastic range 10-30 times greater than that of a normal spring material. This effect is usually observed over a range of about 0-40 K (0-40 °C; 0-72 °F) above the A_{f} temperature.

Also disclosed herein is a process for modulating biological tissue and/or bone, the process comprising: (i) optionally surgically weakening the tissue and/or bone to be treated, such as by making one or more scores to an area of the tissue and/or bone, and (ii) attaching the device of the invention to the tissue and/or bone to be modulated and allowing it to warm to body temperature.

In a further aspect of the invention, there is provided a kit comprising a device according to the invention, and a plurality of pins and/or screws for attaching the device to a section of bone to be modulated.

It will be appreciated that any of the features mentioned above in relation to the device of the invention or the method of producing the device of the invention are also applicable to the process for modulating bone and the kit mentioned above.

The invention will now be described in more detail by way of example only, and with reference to the following figures.

### Figures

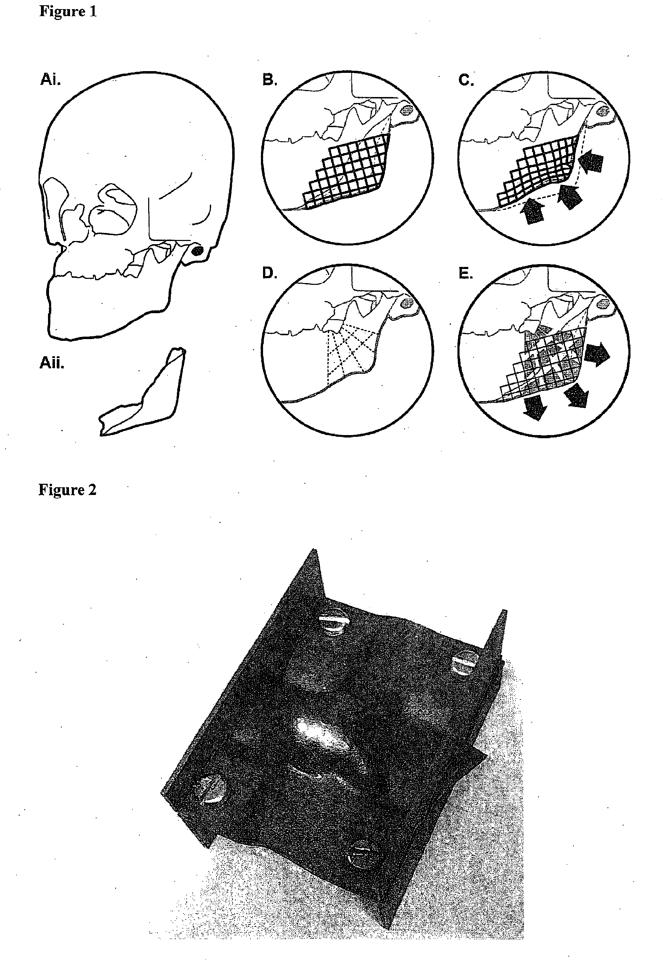

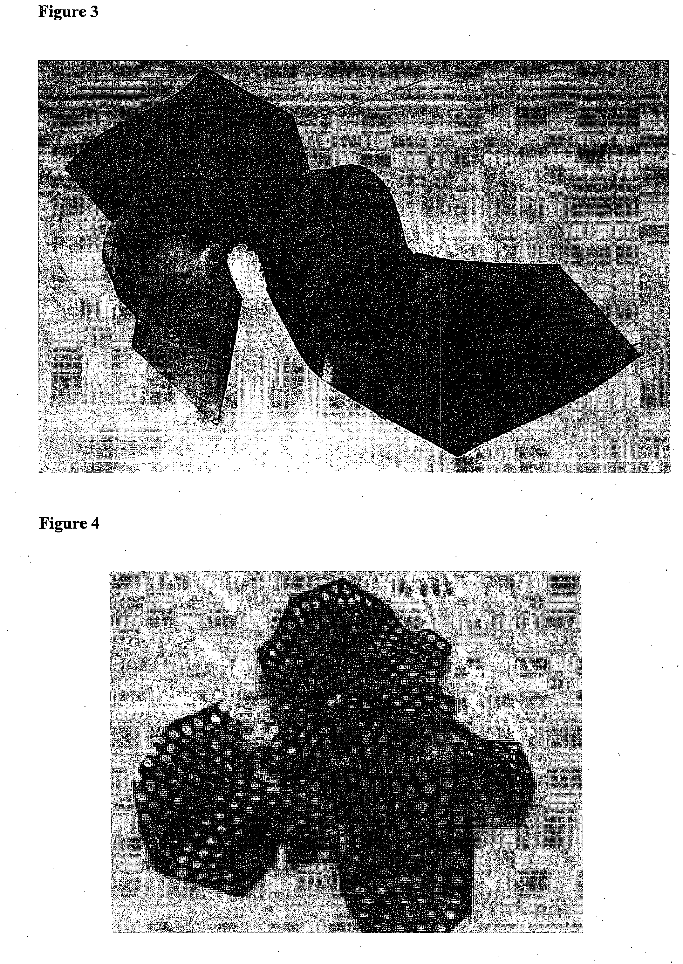

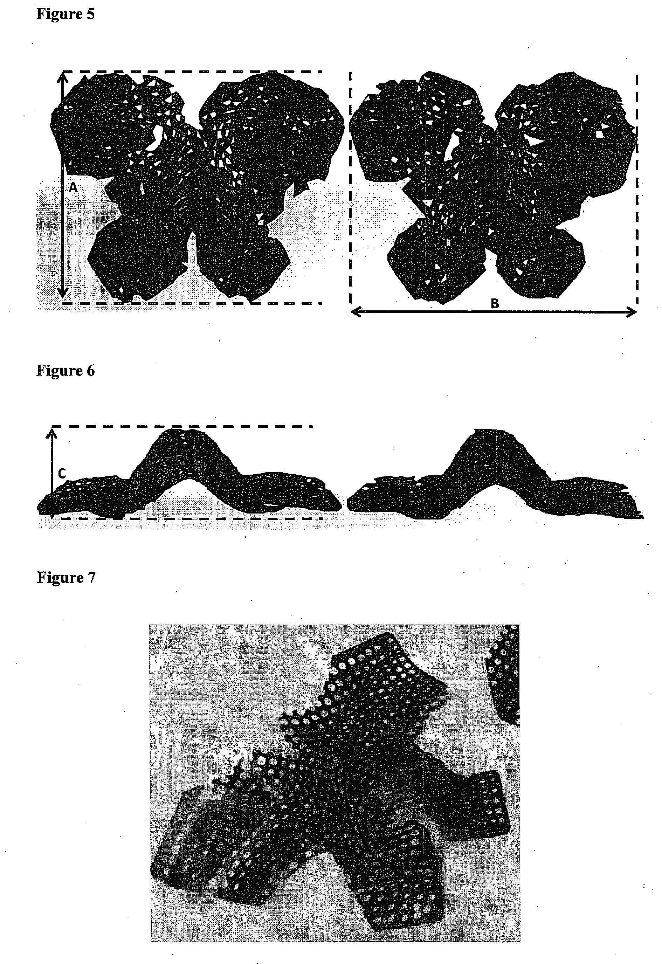

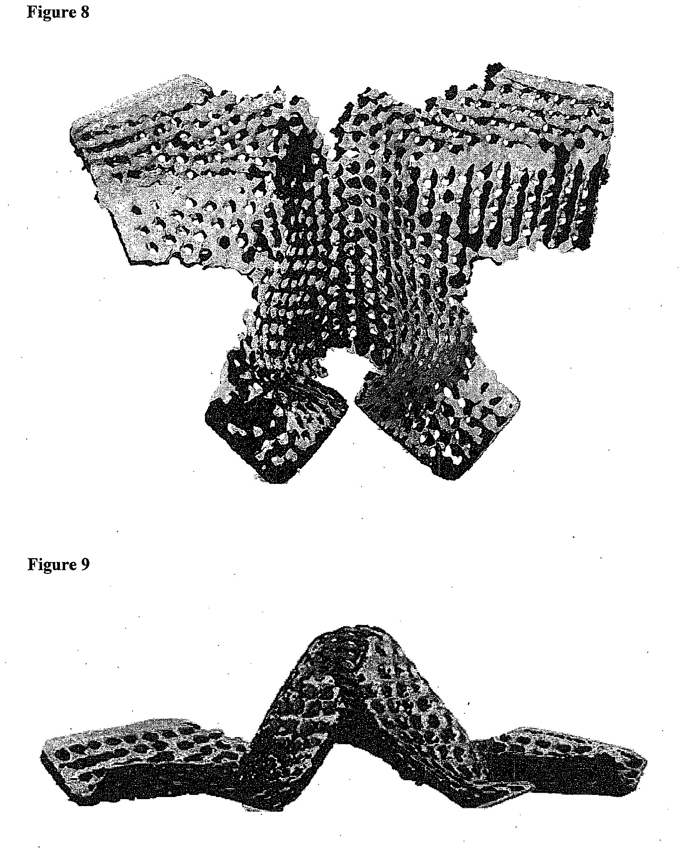

*Figure 1*
   A schematic adjustment to mandibular shape. A 3D CT scan of the skull is made and from this a stereolithiographic model is made to produce an exact replica of the skull (Figure Ai). In the case shown, the angle of the mandible is too small and so a 3D template is made which will alter the mandible to its desired shape (Figure Aii). A memory material mesh is then taken and moulded to the desired shape of the mandible (Figure B). This is then heated to high temperatures so that the memory of the mesh is fixed in this shape. The framework is then cooled, e.g. to room temperature, so that it is in its malleable phase. The mesh is then moulded to the shape of the existing deformed mandibular angle (Figure C). The mesh is then sterilised and prepared for operative use. At operation, the mandible is prepared by performing multiple corticotomies to weaken the bone (Figure D). The mesh is then fixed to the mandible using multiple pins or screws. The access wound is then closed. As the mesh warms to body temperature, it transforms to its pre-programmed shape. This force distracts and moulds the mandible to the planned shape (Figure E).
*Figure 2*
   A nasal mould comprising top and bottom opposing sections, fixed together by means of screws.
*Figure 3*
   A nitinol sheet obtained from the nasal mould of Figure 2 following heating and fixation to the pre-programmed, memory conformation, as described in Example 1.
*Figure 4*
   A nitinol mesh for reconstructing a nasal section of bone, as obtained by the procedure of Example 2.
*Figure 5*
   CT scans (top view) of the nitinol mesh of Figure 4 pre (left image) and post (right image) restoration to the pre-programmed conformation. The image shows dimensions A and B.
*Figure 6*
   CT scans (side view) of the nitinol mesh of Figure 4 pre (left image) and post (right image) restoration to the pre-programmed conformation. The image shows dimension C.
*Figure 7*
   A nitinol mesh for reconstructing a nasal section of bone, as obtained by the procedure of Example 3.
*Figure 8*
   MIMICS reconstructions (top view) of the nitinol mesh of Figure 7 pre (dark colouring) and post (light colouring) restoration to the pre-programmed conformation, with the images overlayed. As for Figure 5, the image shows dimensions A and B.
*Figure 9*
   MIMICS reconstructions (side view) of the nitinol mesh of Figure 7 pre (dark colouring) and post (light colouring) restoration to the pre-programmed conformation, with images overlayed. As for Figure 6, the image shows dimension C.
*Figure 10*
   A CT scan of a pig's head into which a nitinol mesh has been implanted and a memory conformation test conducted.
*Figure 11*
   MIMICS reconstructions (top view) of a nitinol mesh prepared by the procedure of Example 2, pre (dark colouring) and post (light colouring) images overlayed, following the memory test of Example 4 in a pig's head model. As for Figure 5, the image shows dimensions A and B.
*Figure 12*
   MIMICS reconstructions (top view) of a nitinol mesh prepared by the procedure of Example 2, pre (dark colouring) and post (light colouring) images overlayed, following the memory test of Example 4 in a pig's head model. As for Figure 6, the image shows dimension C.
*Figure 13*
   Illustration showing how deformities of the skull can be corrected using shape memory meshes of the invention, in this case the correction of unicoronal synostosis.
*Figure 14*
   Illustration showing how small intraoral devices according to the invention can be used to create additional alveolar bone to enable the insertion of dental implants.
*Figure 15*
   Illustration showing how a shape memory mesh of the invention can be used to correct congenital or post traumatic deformities of bones of the upper and lower limb.
*Figure 16*
   A 3D printed model of a skull having a fusion of the right coronal suture (unicoronal synostosis), which has been modified using modelling clay by a plastic surgeon to reproduce the desired shape of the skull.
*Figure 17*
   A nitinol mesh inside a metal mould for pre-programming of the nitinol mesh into the desired shape of the skull.

### Examples

### Example 1 - Nitinol sheet memory test

The nasal area of a healthy female subject (age 37) was scanned using a 3D scanner (Rodin4D apparatus). The scan was processed and the 3D surface of the nose was extracted. The 3D surface was then processed using CADCAM software in order to create the shape of a mould. Both top and bottom sections of a mould were produced to 2 mm thickness. Digital laser metal sintering (DLMS) was used to rapid prototype the top and bottom moulds.

A hyperelastic nitinol sheet (Ni = 55.74%, Ti = 44.25%; A_{f} = 31.55 °C) was purchased from Johnson Matthey (Royston, UK). A diamond dental saw was used to create a shape approximating the area of the nose to be treated. The piece of nitinol sheet was inserted into the mould, pressed, and the top and bottom sections of the mould were secured together using screws. The mould was then heated to 500 °C in order to set the nitinol sheet into its pre-programmed, memory shape (austenite phase). The product shape was inspected for suitability.

The moulded nitinol shape was cooled to -5 °C in order to convert the material into its malleable martensite phase, and flattened. The flattened shape was retained as long as the temperature remained below the transition temperature. Upon gentle heating, in this case using warm water, the nitinol sheet regained its memory shape (austenite phase).

### Example 2 - Nitinol mesh memory test

The procedure outlined in Example 1 was repeated for a nitinol mesh, which was produced by creating a number of 2 mm holes in a sheet of nitinol (see Figure 4). The mesh was also further refined around the border region of the mesh for improved introduction into the mould.

A CT scan was performed to assess the accuracy of the shape memory test. The results are displayed in Table 1, and show that all dimensions of the mesh were restored to within 2% of the memory shape.
Pre = before shape memory test - a CT scan was performed immediately after the thermal treatment to set the shape.
Post = after the shape memory test.

Scan parameters:
Slice thickness = 0.3 mm;
Pixel spacing = [0.3 mm, 0.3 mm];
Row = 400; and
Column = 400.
The images were processed with MIMICS (level set and region growing segmentation of the image) to create the 3D geometry of the nitinol nasal configuration.

**Table 1.**

| **Dimension** | **Pre (mm)** | **Post (mm)** |
|---|---|---|
| **A** | 99.02 | 98.56 |
| **B** | 76.58 | 75.93 |
| **C** | 29.97 | 30.53 |

### Example 3 - Nitinol mesh memory test

The procedures outlined in Examples 1 and 2 were repeated for a further nitinol mesh, which was produced by creating a number of 2 mm holes in a sheet of nitinol (see Figure 7). This is with the exception that the mesh and mould were heated to 580 °C in order to set the nitinol sheet into its pre-programmed, memory shape (austenite phase).

A CT scan was performed to assess the accuracy of the shape memory test. The results are displayed in Table 2, and show that all dimensions of the mesh were restored to within 2% of the memory shape.
Pre = before shape memory test - a CT scan was performed immediately after the thermal treatment to set the shape.
Post = after the shape memory test.

Scan parameters:
Slice thickness = 0.3 mm;
Pixel spacing = [0.3 mm, 0.3 mm];
Row = 800; and
Column = 400 (800 for pre).
The images were processed with MIMICS (level set and region growing segmentation of the image) to create the 3D geometry of the nitinol nasal configuration.

**Table 2.**

| **Dimension** | **Pre (mm)** | **Post (mm)** |
|---|---|---|
| **A** | 95.40 | 94.33 |
| **B** | 76.06 | 75.44 |
| **C** | 28.56 | 28.19 |

### Example 4 - Pig model memory test

The procedure and nitinol mesh as described in Example 2 was assessed in a pig head model.

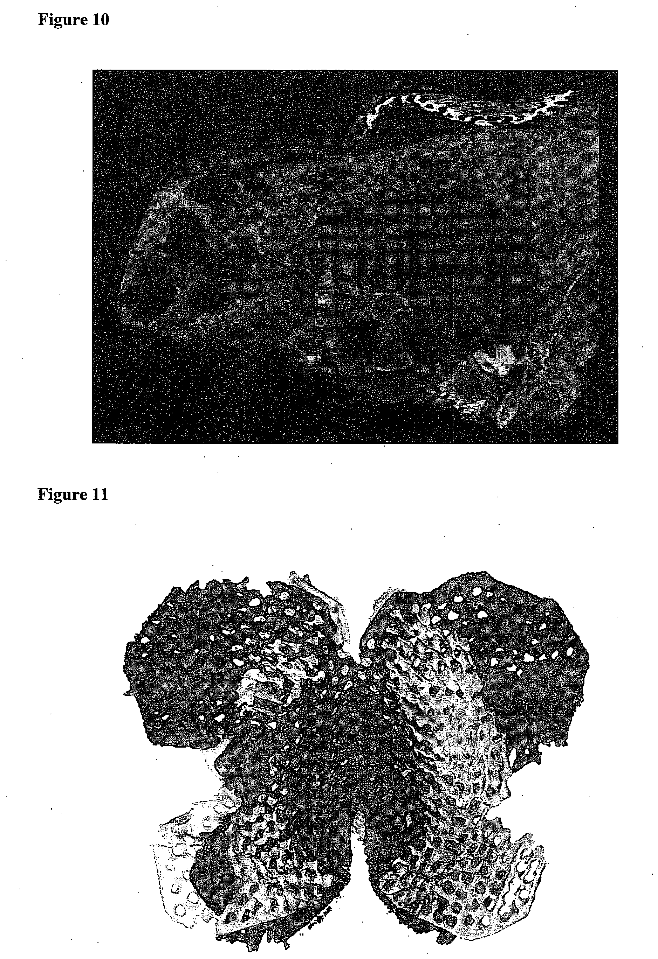

A pig head was obtained from a butcher and the flattened nitinol mesh of Example 2 was implanted (see Figure 10). Due to the temperature of the pig head, the mesh regained its memory shape and was assessed by means of a CT scan (using the same parameters as Example 2). The pre and post images were processed with MIMICS and compared in order to determine the effect imposed by the surrounding tissue of the pig's forehead (see Figures 11 and 12). The results showed that, in the case of the C dimension, the mesh returned to within 39% of the memory shape. Thus, the shape of the surrounding tissue was significantly modified by the memory effect of the device when implanted, thereby promoting tissue remodelling and regeneration.

### Example 5 - Unicoronal synostosis distractor

A nitinol distractor was produced for a patient having developed fusion of the right coronal suture (unicoronal synostosis) by the age of 16 months.

A CT scan was acquired of the whole skull, and a 3D model was created using MIMICS. A 3D printed model of the skull (from skull top to orbits) was produced by means of a rapid prototyping technique. The model was then modified using modelling clay by a plastic surgeon to reproduce the desired shape of the skull (see Figure 16). The modified model was scanned using a 3D scanner and the shape of the remodelled skull was superimposed on the initial anatomy.

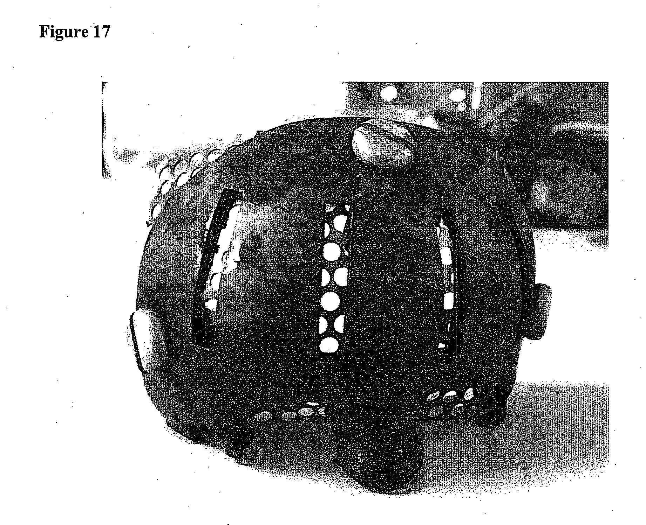

The corrected shape of the skull was used to design a metal mould, which was then produced using metal rapid prototyping by direct laser metal sintering. A nitinol mesh was produced from a shape memory nitinol sheet, inserted into the mould and treated at 500° C for 15 min (see Figure 17). The nitinol mesh was removed from the mould and flattened, and the shape memory effect was tested using hot water (i.e. at or above body temperature). The sheet substantially returned to the pre-programmed shape.

## Claims

1. A device for distracting bone, the device comprising a shape memory material and being capable of distracting bone in 3 dimensions, wherein the shape memory material is nitinol and is a continuous sheet, mesh or web, and **characterized in that** the shape memory material is arranged into a predetermined 3-dimensional conformation in the austenitic phase of nitinol which corresponds to the desired shape of the bone to be distracted.

2. A device according to claim 1, wherein the shape memory material is a mesh or web and comprises a network of geometric shapes.

3. A device according to any preceding claim, for use in bone distraction.

4. A device for use according to claim 3 in calvarial remodeling, including posterior vault expansion, craniosynostosis and/or sagittal synostosis.

5. A process for producing a device for distracting bone, the process comprising:
(i) determining the current and desired conformations of the bone;
(ii) shaping a device comprising a shape memory material into the desired bone conformation at a temperature around or above body temperature;
and
(iii) moulding the device into the current bone conformation at a temperature below body temperature,
wherein the shape memory material is nitinol.

6. A process according to claim 5, wherein the determination of the current conformation of the bone is conducted using computed tomography.

7. A process according to claim 5 or claim 6, wherein the determination of the desired conformation of the bone is conducted using a principal component analysis-derived, computer-generated template.

8. A process according to any one of claims 5 to 7, wherein shaping of the device is conducted at a temperature between the forming temperature of the shape memory material and slightly below the melting point of the shape memory material.

9. A process according to any one of claims 5 to 8, wherein the device comprising a shape memory material is a device according to claim 1 or claim 2.

10. A kit comprising a device according to claim 1 or claim 2, and a plurality of pins and/or screws for attaching the device to a section of bone to be distracted.

## Patentansprüche

1. Vorrichtung zur Knochendistraktion, wobei die Vorrichtung ein Formgedächtnismaterial umfasst und zu einer Knochendistraktion in 3 Dimensionen in der Lage ist, wobei das Formgedächtnismaterial Nitinol ist und ein kontinuierliches Blatt, Gitter oder Gewebe ist, **dadurch gekennzeichnet, dass** das Formgedächtnismaterial in der austenitischen Phase des Nitinols in einer vorgegebenen dreidimensionalen Konformation angeordnet ist, die der gewünschten Gestalt des der Distraktion zu unterziehenden Knochens entspricht.

2. Vorrichtung nach Anspruch 1, wobei das Formgedächtnismaterial ein Gitter oder Gewebe ist und ein Netzwerk aus geometrischen Formen umfasst.

3. Vorrichtung nach einem der vorhergehenden Ansprüche zur Verwendung bei der Knochendistraktion.

4. Vorrichtung zur Verwendung nach Anspruch 3 bei der Kalottenumformung, einschließlich posteriorer Gewölbeexpansion, Kraniosynostose und/oder Sagittalsynostose.

5. Verfahren zur Herstellung einer Vorrichtung zur Knochendistraktion, wobei das Verfahren umfasst:
(i) Bestimmen der gegenwärtigen und gewünschten Konformationen des Knochens;
(ii) Formen einer ein Formgedächtnismaterial umfassenden Vorrichtung zu der gewünschten Knochenkonformation bei einer Temperatur in der Nähe oder oberhalb der Körpertemperatur; und
(iii) Modellieren der Vorrichtung in die gegenwärtige Knochenkonformation bei einer Temperatur unterhalb der Körpertemperatur,
wobei das Formgedächtnismaterial Nitinol ist.

6. Verfahren nach Anspruch 5, wobei das Bestimmen der gegenwärtigen Konformation des Knochens unter Verwendung einer Computertomographie durchgeführt wird.

7. Verfahren nach Anspruch 5 oder Anspruch 6, wobei das Bestimmen der gewünschten Konformation des Knochens unter Verwendung einer mittels Hauptkomponentenanalyse erlangten, computergenerierten Vorlage durchgeführt wird.

8. Verfahren nach einem der Ansprüche 5 bis 7, wobei das Formen der Vorrichtung bei einer Temperatur zwischen der Formungstemperatur des Formgedächtnismaterials und etwas unterhalb des Schmelzpunkts des Formgedächtnismaterials durchgeführt wird.

9. Verfahren nach einem der Ansprüche 5 bis 8, wobei die ein Formgedächtnismaterial umfassende Vorrichtung eine Vorrichtung gemäß Anspruch 1 oder Anspruch 2 ist.

10. Kit umfassend eine Vorrichtung gemäß Anspruch 1 oder Anspruch 2 und eine Vielzahl an Nadeln und/oder Schrauben zum Befestigen der Vorrichtung an einem Abschnitt des der Distraktion zu unterziehenden Knochens.

## Revendications

1. Dispositif de distraction d'os, le dispositif comprenant un matériau à mémoire de forme et étant capable de distraire un os en 3 dimensions, dans lequel le matériau à mémoire de forme est du nitinol et est une feuille continue, un maillage ou une bande, et **caractérisé en ce que** le matériau à mémoire de forme est agencé dans une conformation prédéterminée en 3 dimensions lors de la phase austénitique du nitinol qui correspond à la forme souhaitée de l'os à distraire.

2. Dispositif selon la revendication 1, dans lequel le matériau à mémoire de forme est un maillage ou une bande et comprend un réseau de formes géométriques.

3. Dispositif selon l'une quelconque des revendications précédentes, destiné à être utilisé pour une distraction d'os.

4. Dispositif destiné à être utilisé selon la revendication 3 pour un remodelage de la voûte crânienne, comprenant une extension de voûte postérieure, une craniosynostose et/ou une synostose sagittale.

5. Procédé de fabrication d'un dispositif de distraction d'os, le procédé comprenant :
(i) la détermination de conformations actuelle et souhaitée de l'os ;
(ii) la mise en forme d'un dispositif comprenant un matériau à mémoire de forme dans la conformation d'os souhaitée à une température située autour de ou supérieure à la température corporelle ;
et
(iii) le moulage du dispositif dans la conformation d'os actuelle à une température inférieure à la température corporelle,
dans lequel le matériau à mémoire de forme est du nitinol.

6. Procédé selon la revendication 5, dans lequel la détermination de la conformation actuelle de l'os est effectuée à l'aide de tomographie informatisée.

7. Procédé selon la revendication 5 ou 6, dans lequel la détermination de la conformation souhaitée de l'os est effectuée à l'aide d'un modèle de composants principaux dérivé d'une analyse et généré informatiquement.

8. Procédé selon l'une quelconque des revendications 5 à 7, dans lequel la mise en forme du dispositif est effectuée à une température comprise entre la température de mise en forme du matériau à mémoire de forme et une température légèrement inférieure au point de fusion du matériau à mémoire de forme.

9. Procédé selon l'une quelconque des revendications 5 à 8, dans lequel le dispositif comprenant un matériau à mémoire de forme est un dispositif selon la revendication 1 ou 2.

10. Kit comprenant un dispositif selon la revendication 1 ou 2, et une pluralité de broches et/ou de vis destinées à fixer le dispositif sur une section d'un os à distraire.
